(19) Europäisches Patentamt
European Patent Office
Office européen des brevets

(11) EP 1 132 099 A1

(12) **EUROPEAN PATENT APPLICATION**
published in accordance with Art. 158(3) EPC

(43) Date of publication:
**12.09.2001 Bulletin 2001/37**

(21) Application number: **99972111.1**

(22) Date of filing: **17.11.1999**

(51) Int Cl.[7]: **A61K 48/00**

(86) International application number:
**PCT/JP99/06415**

(87) International publication number:
**WO 00/29031 (25.05.2000 Gazette 2000/21)**

(84) Designated Contracting States:
**AT BE CH CY DE DK ES FI FR GB GR IE IT LI LU MC NL PT SE**

(30) Priority: **18.11.1998 JP 32812698**

(71) Applicant: **HISAMITSU PHARMACEUTICAL CO. INC.**
**Tosu-shi Saga 841-0017 (JP)**

(72) Inventors:
- **GOTO, Takeshi,**
  **Tsukuba Lab.of Hisamit Phar. Co Inc**
  **Tsukuba-shi, Ibaraki 305-0856 (JP)**
- **YONEMURA, K.,**
  **Tsukuba Lab.of Hisamit Phar. Co. Inc**
  **Tsukuba-shi, Ibaraki 305-0856 (JP)**
- **KUWAHARA, T.,**
  **Tsukuba Lab. of Hisamit Phar. Co.Inc**
  **Tsukuba-shi Ibaraki 305-0856 (JP)**
- **OYA, M.,**
  **Tsukuba Lab. of Hisamitsu Pharm. Co. Inc.**
  **Tsukuba-shi, Ibaraki 30 5-0856 (JP)**
- **AKIYAMA, K.,**
  **Tsukuba Lab. of Hisamit Phar. Co. Inc**
  **Tsukuba-shi, Ibaraki 305-0856 (JP)**

(74) Representative: **Grünecker, Kinkeldey, Stockmair & Schwanhäusser Anwaltssozietät**
**Maximilianstrasse 58**
**80538 München (DE)**

(54) **NUCLEIC ACID TRANSPORTERS AND MEDICINAL COMPOSITIONS FOR GENE THERAPY**

(57) A novel nucleic acid carrier and a pharmaceutical composition for gene therapy are disclosed. The nucleic acid carrier of this invention is characterized by containing a polypeptide comprising diaminobutyric acid with a suitable number of residues and/or a pharmaceutically acceptable salt thereof. The nucleic acid carrier of this invention can form a complex with a variety of therapeutic genes that is safe and has extremely low immunogenicity (the pharmaceutical composition of this invention); and it can allow the therapeutic gene to be introduced into cells efficiently and safely whereby high expression of the gene in the cells can be realized.

*Fig.1*

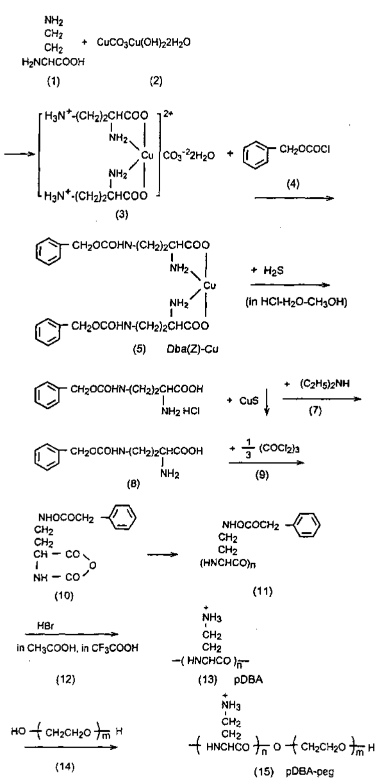


EP 1 132 099 A1

## Description

### Technical Field

This invention relates to nucleic acid carriers for the introduction of therapeutic genes into cells and pharmaceutical compositions for gene therapy containing the nucleic acid carrier and the therapeutic gene. More specifically, this invention' relates to a nucleic acid carrier as well as to a pharmaceutical composition for gene therapy capable of efficiently, safely introducing into cells a therapeutic gene that comprises a nucleic acid and/or a nucleotide derivative which interacts with the nucleic acid carrier, the composition further having a desired effect and, at the same time, allowing the effect to manifest for a prolonged period.

### Background Art

Gene therapy is a totally novel therapeutic method by which an exogenous gene capable of acting as a drug (hereinafter referred to as "therapeutic gene") is introduced (transferred) into the body (in vivo) and is allowed to express for the purpose of performing the treatment of diseases. The diseases for which therapeutic effects can be expected through gene therapy include all those, congenital or acquired, which are caused by the aberrations of gene. Especially, gene therapy is considered a method of treatment that is highly useful for cancers and AIDS which are lethal and for which no cures have been established. Gene therapy is largely classified into "Augmentation Gene Therapy" in which aberrant (causative) genes are left intact and new (normal) genes are augmented and "Replacement Therapy" in which aberrant genes are replaced with normal genes.

For clinical applications of gene therapy, clinical investigations have already begun in Italy, Holland, France, and China since 1989 when the first clinical investigation of gene therapy was conducted in the United States. However, one of the major technical tasks in the clinical applications of gene therapy proves to be the development of methods for and forms of genes optimal for the efficient and safe introduction of therapeutic genes into target cells. In early 1980s physical techniques such as microinjection were applied for use, but they were not able to effect the consistent and efficient introduction of therapeutic genes required for the treatment of diseases. Thus their clinical applications were not realized. Later, recombinant viruses (virus vectors) were developed that serve as vehicles for the efficient introduction of the therapeutic genes into cells; and this has, for the first time, allowed the clinical applications of gene therapy.

The virus vector that currently attracts most attention is a retrovirus vector derived from Molony Murine Leukemia Virus (hereinafter referred to as "MoMLV"), which utilizes the advantages of the life cycle of the virus. Retroviruses posses the property of incorporating their genetic information into genomic DNAs once they have infected the host (Miller D. G. et al., Mol. Cell. Biol., 10,4239, 1990); therefore, they are convenient for the continued expression of therapeutic genes. They are also able to infect various cell species, and thus these many kinds of cells can possibly be the subject of the treatment using the MoMLV vector. On the other hand, this property makes it impossible to administer the virus vector to the body. The wide range of hosts available for the virus namely means that its ability to accumulate within the target cells is poor when administered to the body: systemic administration methods such as intravenous administration cannot be practiced from the standpoint of therapeutic and side effects. Accordingly, the current method of treatment is that a therapy is conducted such that after the target cells are separated from the body, they are subjected to gene introduction in vitro and then returned to the body again ("ex vivo gene introduction" in U. S. Pat. No. 5399346). Although this technique can reliably introduce the therapeutic genes into the target cells and therapeutic effects can also be expected, it requires special equipment for the handling of virus vectors as well as equipment for the large-scale culturing of cells. This limits facilities where the therapy can be performed.

For the foregoing reasons, it is desired that vectors capable of gene introduction in vivo be developed. To have virus vectors produced stably, the production by virus vector-producing cells should be efficient; however, the virus vector-producing cells that are currently under development require a large amount of cells to be cultured to produce the virus vectors in amounts necessary for therapy. Thus, there is a disadvantage that their production proves to be very expensive as compared with that of generic drugs. Furthermore, where the virus vectors are used, they cannot be administered repeatedly because of their high immunogenicity (especially, with Adenovirus vectors) and there are limitations on the sizes of the therapeutic genes, which are disadvantageous.

In addition, parallel efforts are being made to utilize synthetic polyamino acids as the vehicles for gene introduction in place of such virus vectors, to bind the synthetic polyamino acids to drugs for delivery, and to deliver them to or within target cells. WO79/00515 and U.S. Pat. No. 5162505 disclose complexes where polyamino acids and drugs to be delivered (nucleotide analogs, enzymes, etc.) are bonded covalently. However, the means with which the polyamino acid retains the drug is a covalent bond; and this is undesirable where release (separation) of the vehicle from the drug is needed when it is incorporated into the cells. Further, Wu et al. (G.Y.Wu and C.H.Wu, Advanced Drug Delivery Reviews, 12, 159, 1993) prepared a polyamino acid/gene complex in which the polyamino acid, particularly polylysine

serves as vehicle of the gene, and succeeded in the expression of gene by allowing the complex to act on cells and introducing the gene into the cells. Nevertheless, the complexes having polyamino acids, particularly polylysine, as carriers tend to form precipitates with increasing concentrations, and thus they find difficulties in being used in the actual treatment of diseases. Especially when a drug solution containing particles that likely form precipitates in veins is administered, it can be the cause responsible for blockade of blood vessels, thrombus and the like. Even in the case of local administration, there is a problem such as the clogging of syringe needles; and another problem is that it is not possible to introduce into the target cells, a gene in an amount sufficient to carry out treatment.

Therefore, in WO95/09009 a random copolymer of polylysine and serine is shown as a nucleic acid carrier (vehicle) that can form a complex with a therapeutic gene without causing precipitation. However, neither its actual administration to the body nor safety is disclosed explicitly.

S. Ferrari et. al (Gene Therapy, 4, 1100, 1997) reported the efficient introduction of genes with polyethyleneimine. Polyethyleneimine, however, is a substance that is not naturally present in the body, and thus its administration to the body is problematic.

In Japanese Published Patent Application Hei 9-173067, a lipopeptide obtained by attaching an aliphatic acid to 1 to 20 cationic amino acids is used for gene introduction; and it is reported that diaminobutyric acid is most effective as the amino acid to be attached to the aliphatic acid among lysine, ornithine, diaminobutyric acid, and diaminopropionic acid. However, the publication only discloses that a vehicle obtained by attaching an aliphatic acyl group having C10 to C14 to diaminobutyric acid and liposome of dioleoynylphosphatidylethanolaminine were co-administered to cultured cells. In addition to the lack of practicality, there arise problems such as complicated manipulations and high cost in that the vehicle containing diaminobutyric acid and the liposome are used at the same time.

Accordingly, at present there is a strong need for the research and development in nucleic acid carriers capable of being safely and efficiently introduced into the body and of sufficiently exerting the effects of therapeutic genes, as well as in pharmaceutical compositions for gene therapy using said nucleic acid carriers.

## Disclosure of the Invention

It is an object of this invention to provide a nucleic acid carrier for the efficient and safe introduction of a therapeutic gene into cells. It is also an object of the invention to provide a pharmaceutical composition for gene therapy capable of efficiently, safely introducing into cells a therapeutic gene.

As a result of having pursued diligent investigations to solve the above-stated problems, the present inventors found that when a vehicle comprising diaminobutyric acid and/or a pharmaceutically acceptable salt thereof was used as a nucleic acid carrier for carrying a therapeutic gene, the therapeutic gene could be introduced into cells efficiently and safely and the functions of nucleic acids or the like in the introduced gene were allowed to manifest for a prolonged period. Thus, this invention has been accomplished. As used herein, the term, "safely" means no formation of insoluble precipitates, low antigenicity or the like.

Specifically, this invention provides a nucleic acid carrier comprising a polypeptide comprising a basic amino acid and/or a pharmaceutically acceptable salt thereof.

Also, the invention provides a nucleic acid carrier comprising a polypeptide comprising diaminobutyric acid and/or a pharmaceutically acceptable salt thereof.

Further, the invention provides a nucleic acid carrier comprising a block copolymer of a polypeptide comprising diaminobutyric acid and/or a pharmaceutically acceptable salt thereof and polyethylene glycol.

Still further, the invention provides a nucleic acid carrier as described above, wherein the polypeptide comprises diaminobutyric acid and/or a pharmaceutically acceptable salt thereof, each having a residue number of from 20 to 280.

Also, the invention provides a pharmaceutical composition for gene therapy comprising any of the nucleic acid carriers described above and a therapeutic gene.

Also, the invention provides a pharmaceutical composition for gene therapy as described above capable of specifically expressing the therapeutic gene in liver when systemically administered.

Further, according to the invention, there is provided a pharmaceutical composition for gene therapy as described above capable of sustaining good continued efficacy when systemically administered, wherein the efficacy continues at least for 50 to about 210 days.

Also, the invention provides a pharmaceutical composition for gene therapy as described above wherein the therapeutic gene is any of a variety of nucleic acids (including those derived from natural products and those chemically synthesized), or any of a variety of nucleotide derivatives (including those chemically modified).

Further, the invention provides a pharmaceutical composition for gene therapy as described above wherein the nucleotide derivative is an antisense (oligonucleotide) or TFO (Triplex Forming Oligonucleotide).

Also, the invention provides a pharmaceutical composition for gene therapy as described above, said composition having low immunogenicity.

The pharmaceutical composition for gene therapy according to the invention may further contain other components if necessary.

Further, the pharmaceutical composition for gene therapy according to the invention may contain a nucleic acid carrier linked by a variety of ligands capable of specifically recognizing tissues for the purpose of tissue-specific incorporation.

## Brief Description of the Drawings

Fig. 1 is a representation showing an example of the synthetic pathway for the nucleic acid carrier of this invention.

Fig. 2 shows the results from measurement of the activity of the expressed luciferase when measured in Example 2, where "12," "26," "49," "62," "170," and "348" respectively represent pDBAs having MW (molecular weight) of 1,900, 4,200, 7,800, 9,900, 27,200, and 55,700 which were respectively obtained in Synthetic Examples Nos. 1, 2, 3, 4, 5, and 7 in Table 2.

Fig. 3 shows the results from measurement of the activity of the expressed luciferase when measured in Example 3, where pDBA represents that obtained in Synthetic Example 3 in Table 2 and pDBApeg represents that obtained in Synthetic Example 8.

Fig. 4 is a graph showing the results from measurement of the activity of the expressed luciferase when measured in Example 4, where "non-T.f." represents non-transfection and "12," "26," "49," "62," and "170," respectively represent pDBAs having MW (molecular weight) of 1,900, 4,200, 7,800, 9,900, and 27,200 which were respectively obtained in Synthetic Examples Nos. 1, 2, 3, 4, and 5 in Table 2.

Fig. 5 is a graph showing the results obtained in Example 5. Fig 5A represents those after two days, while Fig. 5B represents those after 21 days. In the figures, pDBA represents that obtained in Synthetic Example 3 in Table 2.

Fig. 6 is a graph showing the results obtained in Example 6.

Fig. 7 is a graph showing the results obtained in Example 7, where "12," "26," "49," "62," and "170," respectively represent pDBAs having MW (molecular weight) of 1,900, 4,200, 7,800, 9,900, and 27,200 which were respectively obtained in Synthetic Examples Nos. 1, 2, 3, 4, and 5 in Table 2.

Fig. 8 is a graph showing the results obtained in Example 8, where pDBA represents that obtained in Synthetic Example 3 in Table 2.

Fig. 9 is a graph showing the results from observation of the symptoms appearing up to one hour after intravenous administration as obtained in Example 10.

Fig. 10 is a graph showing the results from measurement of the diameters of tachetic pigmentation appeared 30 minutes after intravenous administration as obtained in Example 10.

Fig. 11 is a graph showing the results from the measurement with a flow cytometer as obtained in Example 11. Fig. 11A shows those obtained when only FITC Oligo was used as control. Fig. 11B shows those obtained when FITC Oligo and pDBA#49 (with MW(7,800) as obtained in Synthetic Example 3 in Table 2) were used. Fig. 11C shows those obtained when FITC Oligo and pDBA#170 (with MW(27,200) as obtained in Synthetic Example 5 in Table 2) were used.

Fig. 12 is a graph showing the results from evaluation of the ratios of the introduced cells as obtained in Example 11, where "12," "49," and "170," respectively represent pDBAs having MW (molecular weight) of 1,900, 7,800, and 27,200 which were respectively obtained in Synthetic Examples Nos. 1, 3, and 5 in Table 2.

## Best Mode for Carrying out the Invention

(Structures of Nucleic Acid Carriers)

The structure possessed by a nucleic acid carrier of this invention is a polypeptide comprising diaminobutyric acid and includes the structure represented by formula (1) wherein "n" represents a natural number.

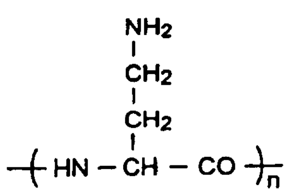

(1)

The other structure possessed by a nucleic acid carrier of this invention is a polypeptide comprising a pharmaceutically acceptable salt of diaminobutyric acid and includes the structure represented by formula (2) wherein "n" represents a natural number.

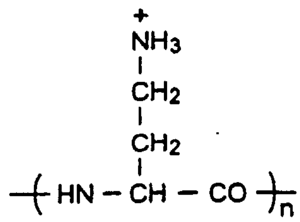

(2)

Further, the nucleic acid carrier of this invention may be a structure including the two structures described above in any proportion. Namely, embraced is that existing in the form of diaminobutyric acid or a salt thereof in any proportion.

Still further, in the diaminobutyric acid D-form and L-form can exist which are optical isomers; and the nucleic acid carrier of this invention may also include a polypeptide which consists of D-form, L-form or a mixture thereof in any proportion of diaminobutyric acid. In other words, the carrier may be a polypeptide containing any of poly-L-diaminobutyric acid, poly-D-diaminobutyric acid, and poly-DL-diaminobutyric acid.

The residue of the nucleic acid carrier according to this invention refers to diaminobutyric acid (or a salt thereof) residue which is a monomer forming the polypeptide; and the number of residues refers to the number of the molecules (represented by "n" in the above formulae). The preferred number of residues of the nucleic acid carrier according to this invention is at least 10 residues. More preferably, the number of residues is at least 20 residues, and most preferably at least 25 residues. The preferred number of residues of the nucleic acid carrier according to this invention is 280 residues or less. More preferably, the number of residues is 250 residues or less. When the number of residues is too large, the synthesis will be difficult and handling will also be inconvenient. On the other hand, when the number of residues is too small, performance as a nucleic acid carrier will be inadequate. Further, one skilled in the art can readily and suitably select the preferred number of residues depending on the therapeutic gene to be used and the properties of components that can be used concurrently.

The diaminobutyric acid salt is not particularly limited insofar as it is a pharmaceutically acceptable salt. Preferably mentioned are, among others, inorganic acids such as hydrochloric acid, sulfuric acid, nitric acid, and phosphoric acid and organic acids such as acetic acid, propionic acid, citric acid, lactic acid, oxalic acid, succinic acid, tartaric acid, malonic acid, fumaric acid, and malic acid. Among these acetate is particularly preferred. Assuming that the polypeptide of the nucleic acid carrier is polydiaminobutyric acid acetate, the number of residues described above can possibly be expressed in terms of molecular weight because the polydiaminobutyric acid acetate has a molecular weight of 160.

The other form of the nucleic acid carrier of this invention includes one having a block copolymer structure where polyethylene glycol is linked to the polypeptide comprising diaminobutyric acid described above and having formula (3) wherein "n" and "m" represent a natural number, respectively.

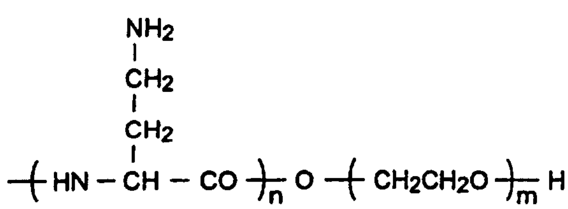

(3)

Further, since the polypeptide has a carboxylic acid group, a copolymer represented by formula (4) wherein "n," "n'," and "m" represent a natural number, respectively is possibly one of those having the block copolymer structure.

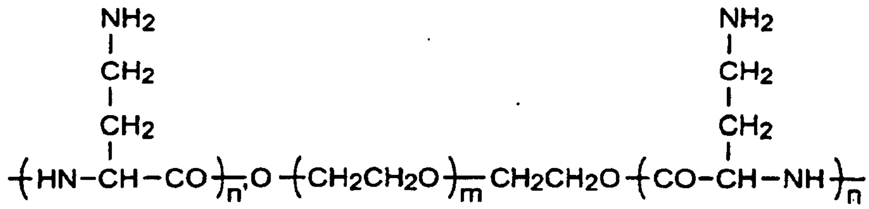 (4)

As used herein, the molecular weight of ethylene glycol (or number "m") is not particularly limited, but it is normally on the order of from 200 to 25,000 for the reasons described later.

(Synthetic Methods for Nucleic Acid Carriers)

There is no particular limitation to the method for synthesizing nucleic acid carriers of this invention that are characterized by having the structures described above. Preferably, usable are organic chemical reactions including a variety of synthetic methods for polypeptides that are ordinarily known (New Experimental Chemistry Monograph No. 19 Polymer Chemistry I, published in 1980, Maruzen Co. Ltd.). More specifically, it is preferred in this invention that the nucleic acid carrier is produced by polymerizing monomer components through a suitable reaction. In this case, the monomers to be used may preferably employ diaminobutyric acid, diaminobutyric acid where a protective group has been introduced into the γ-amino group, and diaminobutyric acid having activated amino groups and/or carboxylic acid groups for peptide group formation. Especially, in this invention it is preferred that the γ-amino group of diaminobutyric acid is protected and the diaminobutyric acid is then converted to an acid anhydride thereof in order to render the peptide bond formation easy (New Experimental Chemistry Monograph No. 19, Polymer Chemistry I, published in 1980, Maruzen Co. Ltd.).

In order to polymerize such monomers, it is possible to use various kinds of initiators in appropriate quantities. Concretely, various amine and alcohol compounds are usable. The amine compounds include alkylamines; particularly, butylamine is preferable. When polyethylene glycols are used as alcohols, the nucleic acid carriers of this invention may be obtained that have polyethylene glycol moieties attached.

For the synthesis of polypeptides according to this invention, the particularly preferred synthetic pathway is illustrated in Fig. 1. Specifically, it is preferred that the γ-amino group of diaminobutyric acid is protected by an appropriate protecting group and the resulting protected amino acid is converted, using phosgene, to an amino acid anhydride in order to use it as a monomer in polycondensation ((10) in Fig. 1). Employing this monomer, the polycondensation reaction allows suitable initiators to be used; and it becomes possible to introduce a preferable number of monomers. Although the initiator is not particularly limited, various amine compounds are preferable, and the use of butylamine is particularly preferable. When suitable ethylene glycols are used as initiators, it will be possible to obtain block copolymers having ethylene glycol moieties which are polypeptides having preferable numbers of monomers. In practice, the methods as described in Helv. Chim. Acta, 43, 270 (1960) or in New Experimental Chemistry Monograph No. 19, Polymer Chemistry I, published in 1980, Maruzen Co. Ltd. may be followed, or modified to be carried out.

(Detection of Nucleic Acid Carriers)

The structures of the nucleic acid carriers of this invention have the characteristics described previously. Therefore, it will be possible to detect the nucleic acid carriers of the invention based on such structural characteristics. Even if the nucleic acid carriers of this invention themselves or pharmaceutical compositions for gene therapy using them are used in various forms, the detection of nucleic acid carriers of this invention will be likewise possible when suitable pretreatment is performed. One skilled in the art can readily select the necessary treatment.

The method of detection is not particularly limited; and a variety of ordinarily known polypeptide analysis methods can be used (J. Controlled Release 54, 39-48, 1998). Concretely usable are the qualitative and quantitative analysis of diaminobutryic acid, the determination of the number of residues based on molecular weight measurement using various kinds of liquid chromatography, various spectral analyses for detecting the presence of polyethylene glycol groups (infrared absorption spectroscopy and nuclear resonance absorption spectroscopy), mass spectroscopy, and chemical qualitative analysis methods. (Pharmaceutical Compositions for Gene Therapy and Therapeutic Genes)

The pharmaceutical composition for gene therapy according to this invention at least contains the nucleic acid carrier of the invention and a therapeutic gene. As used herein, the therapeutic gene refers to various nucleic acids and/or nucleotide derivatives.

The nucleic acid carriers and the therapeutic genes form complexes at different ratios and can be prepared

into pharmaceutical compositions for gene therapy. When the therapeutic gene/nucleic acid carrier (weight (W)/weight (W)) is in the range of from 2/1 to 1/50, efficacious effects can be obtained. Preferably, when the therapeutic gene/nucleic acid carrier (W/W) is in the range of from 1/1 to 1/30, more efficacious effects can be obtained. If the therapeutic gene/nucleic acid carrier (W/W) is 1/50 or more, it will be undesirable since the amount of free nucleic acid carrier that is not involved in the complex with the therapeutic gene increases. On the other hand, if the therapeutic gene/nucleic acid carrier (W/W) is 2/1 or less, it will be undesirable since the lowered affinity to the surfaces of the cells to be introduced causes the efficiency of introduction of the therapeutic gene to be decreased.

Because the nucleic acid carrier of this invention can bear a positive charge, it is able to retain a therapeutic gene (e.g., nucleic acid) principally having a negative charge through electrostatic bonding. Thus, after it has been delivered to the target cell or within the cell, it effectively releases the therapeutic gene, enabling the expression of the gene. This action can be regulated by appropriately selecting the charge ratio of therapeutic gene/nucleic acid carrier. In this invention, for example, when the ratio is in the range of 1/1 to 1/40, it will produce greater effects. In the case of the charge ratio being 1/1 or less, the lowered affinity to the cell surface causes the efficiency of introduction of the therapeutic genes to be decreased; whereas, the charge ratio of 1/40 or more will be undesirable since the amount of free nucleic acid carrier that is not involved in the complex with the therapeutic gene increases.

There are no particular limitations to the kind, the molecular weight, and the shape of nucleic acid and/or nucleotide derivative of the therapeutic gene to be introduced to cells by the pharmaceutical composition for gene therapy according to this invention, as well as to the sequences of genes to be encoded by the foregoing. Specifically, the molecular weight of nucleic acid is not particularly limited in that it may be from an oligonucleotide with about 20 bases to a cosmid gene with several tens of kilo bases. For the shape of nucleic acid, a single-stranded gene, a double-stranded gene, a triple-stranded forming gene, DNA, RNA, a DNA/RNA chimera-type gene, a phosphothioate-type gene, a straight-chain gene, a circular gene, and the like may be used without any restriction. The sequence of gene to be encoded can employ, in addition to the therapeutic gene, any sequence from a promoter or enhancer for the transcription of the therapeutic gene, a poly-A signal, a marker gene for labeling and/or selecting the cell into which the gene has been introduced, a virus-derived gene sequence for the efficient insertion of gene into cellular genomic DNA sequences, and a signal sequence for extracellularly secreting the substance that acts as drug and/or for having the substance remained at localized sites within a cell.

For the therapeutic genes, genes corresponding to disorders, namely genes that act against the disorders in an antagonistic manner or genes that supplement those lacking in the disorders may be used. For example, mentioned are SOD, anti-inflammatory cytokines, and genes encoding the peptides that act on cell-adhesion factors in an antagonistic manner for inflammatory disorders; genes encoding normal enzymes are mentioned for enzyme-deficient disorders; genes encoding normal receptors are mentioned for receptor-deficient disorders; mentioned for virus infections are thymidine kinases that kill virus-infected cells, genes encoding toxins such as diphtheria toxin and genes encoding antisense, triplehelixes, ribozymes, decoys, and transdominant mutants each of which inhibits the replication of viruses; mentioned for cancers are thymidine kinases that kill cancer cells, genes encoding toxins such as diphtheria toxin, genes encoding antisense, triplehelixes, and ribozymes each of which inactivates cancer genes, cancer-suppressing genes such as p53 that normalize the cancer cells, genes encoding antisense, triplehelixes, and ribozymes each of which inactivates genes that are involved in the multi-drug resistance against anti-cancer agents; and mentioned for familial hypercholesterolemia are genes encoding LDL receptors.

As for the expression cassettes to be used for therapeutic genes, any cassettes without any particular limitations may be used insofar as they can cause genes to express in the target cells. One skilled in the art can readily select such expression cassettes. Preferably, they are expression cassettes capable of gene expression in the cells derived from an animal, more preferably, expression cassettes capable of gene expression in the cells derived from a mammal, most preferably expression cassettes capable of gene expression in the cells derived from a human. The gene promoters that can be used as expression cassettes include: for example, virus-derived promoters from an Adenovirus, a cytomegalovirus, a human immunodeficiency virus, a simian virus 40, a Rous sarcoma virus, a herpes simplex virus, a murine leukemia virus, a sinbis virus, a Sendai virus, a hepatitis type A virus, a hepatitis type B virus, a hepatitis type C virus, a papilloma virus, a human T cell leukemia virus, an influenza virus, a Japanese encephalitis virus, a JC virus, parbovirus B19, a poliovirus, and the like; mammal-derived promoters such as albumin and a heat shock protein; and chimera type promoters such as a CAG promoter.

The signal sequences for extracellularly secreting the drugs that have been encoded by the therapeutic genes and/or for having the drugs remained at localized sites within the cell can employ signal peptides derived from interleukin 2 which assist the extracellular secretion (Fusao Komada, The Abstract of the 115$^{th}$ Annual Meeting of the Pharmaceutical Society of Japan 4, 12, 1995), peptides derived from Adenovirus E1a that promote nuclear localization, peptides derived from polyoma virus large T antigen, peptides derived from SV40 large T antigen, peptides derived from nucleoplasmin, and peptides derived from regulator proteins after transcription of HTLV1p24 (Kalderon, D., et al., Cell, 39, 499, 1984).

The pharmaceutical composition for gene therapy according to this invention may be prepared by mixing with

the nucleic acid carrier, the therapeutic gene that has been designed for therapy as described above. More specifically, after the nucleic acid carrier and the therapeutic gene that has been designed for therapy are respectively dissolved in an appropriate solvent such as water, physiological saline water, and an isotonicated buffer, they are mixed and allowed to stand for 10-30 minutes, thus enabling the preparation. Here, the ratio of the nucleic acid constituting the therapeutic gene to the nucleic acid carrier is not limited, but the nucleic acid carrier may be used in a proportion of from about 0.5 to about 50 μg per μ g nucleic acid, preferably in a proportion of from about 1 to about 30 μg per μg nucleic acid.

(Methods of Use of the Pharmaceutical Composition for Gene Therapy)

The pharmaceutical composition for gene therapy according to this invention can be used in the gene therapy through autologous implantation (ex vivo gene therapy) where the target cells are removed outside the body from the patient and the cells are then returned to the body of the patient after the objective therapeutic gene has been introduced into the cells. The therapeutic gene can also be used in the gene therapy where the therapeutic gene is directly administered to the patient (in vivo gene therapy).

Gene therapy is largely classified into "Augmentation Gene Therapy" in which aberrant (causative) genes are left intact and new (normal) genes are augmented and "Replacement Therapy" in which aberrant genes are replaced with normal genes. The pharmaceutical compositions for gene therapy according to this invention can be used in both therapies.

Methods for administering the pharmaceutical composition for gene therapy according to this invention to the body are not particularly limited and may preferably be carried out by, for example, parenteral administration, administration through injection.

The dosages for the pharmaceutical composition for gene therapy according to this invention differ depending on the intended methods, the intended objects, etc., and one skilled in the art can readily perform appropriate selection and optimization. Where administration by injection is used, preferably administration is done in a daily dose of from about 0.1 μg/kg to about 1000 mg/kg, and more preferably in a daily dose of from about 1 μg/kg to about 100 mg/kg.

The nucleic acid carrier of this invention does not form precipitates when it forms a complex with a therapeutic gene. Therefore, when the pharmaceutical composition for gene therapy is directly administered to the blood vessels, there is no danger of thrombosis formation, and it will be possible to efficiently and precisely administer the therapeutic gene.

The nucleic acid carrier and the pharmaceutical composition for gene therapy according to this invention do not exhibit antigenicity that is normally observed when the therapeutic gene is administered to the body. Thus, repeated administration at certain intervals will be possible to maintain the in vivo therapeutic effects.

The pharmaceutical compositions for gene therapy according to this invention have excellent effects on body tissues such as kidney, spleen, lung, bronchi, heart, liver, brain, nerves, muscle, marrow, small intestine, colon, large intestine, skin, angioendothelium, etc. Particularly, in the case of systemic administration such as intravenous administration, specific incorporation to the liver takes place. Therefore, it will be possible to have the therapeutic gene expressed safely and effectively in the liver.

Furthermore, when the effective tissue-specific incorporation is intended, tissue-specific ligands may be linked to the nucleic acid carrier. For example, when the liver is targeted, a sugar (glactose, lactose, acyloglycoprotein, oligoglactose, hyaluronic acid, etc.) is linked to the nucleic acid carrier with the result of increased affinity to the liver, and more effectiveness will be realized. In order to reduce cytotoxicity, to improve the ability to stay in blood, and to further improve solubility in solvent, it is possible to form a block copolymer of diaminobutyric acid (in the nucleic acid carrier) and polyethylene glycol (PEG), whereby modification can also be achieved: its synthetic method has already been described. In this instance, PEG having a molecular weight of 200 or more can be used. PEG having a molecular weight of 1000 or more is preferable. The molecular weight of PEG is preferably 25,000 or less, more preferably 10,000, and most preferably 5,000 or less. If the molecular weight of PEG is 25,000 or more, affinity to the cell surface decreases, thus resulting in lowered efficiency of nucleic acid introduction; if the molecular weight is 200 or less, the intended effect by polyethylene glycol will be small, which is undesirable.

The pharmaceutical compositions for gene therapy according to this invention allow the expression of therapeutic genes to prolong in the cells in vivo after systemic administration. Specifically, it is possible to cause expression for from 50 days to about 210 days. This prolonged time will possibly be adjusted by appropriately selecting the nucleic acid carrier of this invention. Accordingly, it is very beneficial in that not only the administration proves to be easy, but also the burden on the patient during administration lessens.

This invention will be described more concretely by referring to the examples; however, the invention is not to be limited by these examples.

## EXAMPLES

The synthesis of the nucleic acid carriers used in the examples below was carried out according to the method of K. Vogeler et al. (Helv. Chim. Acta, 43, 270 (1960)). The synthetic pathway is shown in Fig. 1.

In the present specification, polydiaminobutyric acid (poly(2,4-diaminobutyric acid)) and polydiaminobutyric acid acetate are abbreviated as "pDBA." The pDBA is defined to encompass those based on all possible optical isomers. Likewise block copolymers with polyethylene glycol are abbreviated as "pDBApeg" (or pDBA-PEG).

(Example 1) Synthesis of Nucleic Acid Carriers

(I) Synthesis of N-γ-carbobenzoxy-DL-diaminobutyric acid NCA (4N-carbobenzoxy-DL-2,4-diaminobutyric acid N-carboxyanhydride (10) as a monomer

(I-1) Synthesis of N-γ-carbobenzoxy-DL-diaminobutyric acid (4N-carbobenzoxy-DL-2, 4-diaminobutyric Acid) (8):

DL-2,4-diamino-n-butyric acid dihydrochloride (1)(15 g, Sigma-Aldrich Corporation) was dissolved in 75 ml of water. To this was added basic cupper carbonate (2) (11.7 g), and it was allowed to stand. Then it was boiled at reflux and subsequently, it was filtered. Sodium bicarbonate (16.6 g) and carbobenzoxyl chloride (4) (17.8 ml, Wako Pure Chemical Industries, Ltd.) were added to the filtrate and stirred to obtain product as a precipitate. The resulting product was filtered and washed with acetone and diethyl ether. Then drying produced 15 g of N-γ-carbobenzoxy-DL-diaminobutyric acid cupper complex (4N-carbobenzoxy-DL-2,4-diaminobutyric acid copper complex (hereinafter referred to as "Dba(Z)-Cu") (5).

The resulting complex (5) was added to a mixed solution of 35% HCl (19.3 ml), water (22 ml) and methanol (11 ml) and stirred in the presence of hydrogen sulfide ($H_2S$) gas. After allowing the solution to stand at room temperature, excessive hydrogen sulfide was removed and then insoluble materials were removed by filtration. The filtrate was cooled with addition of water and methanol under reduced pressure. After further addition of methanol, the pH of the solution was adjusted to 7 by addition of diethylamine (7). The precipitated crystals were separated by filtration and washed on a filter funnel with diethyl ether. After drying, 4g of product was obtained as a white crystal. Further, the filtrate was concentrated to precipitate crystals. The crystals were separated by filtration and washed on the filter funnel with diethyl ether. After drying, 1.5 g of product was obtained as a white crystal. These crystals were combined to yield a total of 5.5 g of N-γ-carbobenzoxy-DL-diaminobutyric acid (8) (31% yield calculated from the starting amino acid).

(I-2) Synthesis of N-γ-carbobenzoxy-DL-diaminobutyric acid NCA (4N-carbobenzoxy-DL-2,4-diaminobutyric acid N-carboxy-anhydride (10)):

The compound obtained above (8) (5 g) was dissolved in tetrahedrofuran (THF, 200 ml). To this was added 4.5 g of triphosgene (9) (bis(tricholoromethyl)carbonate available from Sigma-Aldrich Corporation) dissolved in 40 ml of THF, and it was stirred at 40 °C for 60 minutes. After removal of solvent under reduced pressure, hexane was added to the resulting crude product for dissolution, after which it was cooled. After further removal of hexane thoroughly under reduced pressure, ethyl acetate was added to the resulting product for dissolution, and the insoluble materials were removed by filtration. When the resulting filtrate was cooled after addition of hexane, product was precipitated as a white crystal. The precipitated crystals were separated by filtration and dried under reduced pressure. The filtrate was concentrated under reduced pressure, and then it was similarly treated to produce product as a crystal. The obtained crystals were recrystallized from diethyl ether to produce 2.7 g (50% yield) of the purified product (10).

(II) Polymerization

Nucleic acid carriers having various numbers of residues were obtained by subjecting N-γ-carbobenzoxy-DL-diaminobutyric acid NCA (10) to condensation polymerization using initiators in different proportions and thereafter, by deprotecting the protecting groups for the amino groups.

As used herein, the number of residues according to this invention refers to that calculated following the equation below (Arieh Yaron et al., Biochim. Biophys. Acta, 69, 397-399, 1963): multiplication by 0.9 at the end of equation has such reason that the molecular weight decreases about 10% under the reaction conditions for removing protective groups. Further, the molecular weights in this invention refer to those expressed by the following equation:

The number of residues=the degree of polymerization=[the quantity of N-γ-carbobenzoxy-DL-diaminobutyric acid NCA (10)(mole number)/the quantity of initiator (mole number)] x yield (%)/100 x 0.9

Molecular weight = n (the degree of polymerization) x quantity of residue (the quantity of residue for DBA acetate as acetate = 160)

Synthetic Example 3 (the number of residues=49) in Tables 1 and 2, which is synthetic examples for poly-DL-diaminobutyric acid (poly(DL-2,4-diaminobutyric acid) and its acetate, is described below. Employing other conditions shown in Table 1, pDBAs in Synthetic Example 1 (the number of residues=12), Synthetic Example 2 (the number of residues=26), Synthetic Example 4 (the number of residues=62), Synthetic Example 5 (the number of residues=170), Synthetic Example 6 (the number of residues=278), and Synthetic Example 7 (the number of residues=348) were obtained similarly. Synthetic conditions for polydiaminobutyric acid-PEG (15) obtained by linking polyethylene glycol (14) (PEG, molecular weight=1000) to polydiaminobutyric acid and its acetate of Synthetic Example 3 are shown as Synthetic Example 8 in Tables 1 and 2.

(II-1) Synthesis of poly-N-γ-carbobenzoxy-DL-diaminobutyric acid (poly(4-N-carbobenzoxy-DL-2,4-diaminobutyric acid) (11):

N-γ-carbobenzoxy-DL-diaminobutyric acid NCA (10) (1 g, 3.6 mmol) was dissolved in 19 ml of acetonitrile, to which butylamine (4.38 mg, 0.06 mmol) was added as an initiator. The solution was allowed to stand at 30 °C for 307 hours. The resulting polymer was filtered and washed with acetonitrile. After extraction with diethyl ether using a Soxlet extractor, it was dried under reduced pressure to produce 0.77 g of poly-N-y-carbobenzoxy-DL-diaminobutyric acid (11) (the degree of polymerization=91%).

(II-2) Synthesis of poly-DL-diaminobutyric acid (poly(DL-2,4-diaminobutyric acid) acetate (13):

Poly-N-γ-carbobenzoxy-DL-diaminobutyric acid (11) (0.5 g) was dissolved in 2 ml of trifluoroacetic acid, to which 25% hydrogen bromide acetate solution (12) was added and mixed with shaking. After allowing the solution to stand, diethyl ether was added thereto and the supernatant ether phase was removed by decantation. The same manipulation was carried out with diisopropyl ether and the supernatant ether phase was removed by decantation. The resulting precipitates were sufficiently brought to dryness under reduced pressure. Sodium acetate and water were added to the obtained solids to produce a mixed solution. The mixed solution was dialyzed with running water using a dialysis tube that would remove materials with a molecular weight of 1000 or less. Then, centrifugation was carried out at 20,000G for 1 hour to remove precipitates. The resulting solution was lyophilized to produce 0.34 g of poly-DL-diaminobutryic acid acetate (13)(yield 91%).

【table 1】

Synthetic Conditions for Poly(4-N-carbobenzoxy-DL-2,4-diaminobutyric acid) by Polycondensation of 4-N-Carbobenzoxy-DL-2,4-diaminobutyric Acid NCA

| Synthetic Example | NCA g(mmol) | Initiator a) mg(mmol) | A/I b) | Solvent c) (ml) | Monomer (mol/l) | Temperature Time | Yield |
|---|---|---|---|---|---|---|---|
| 1 | DL-(Z)diaminobutyric acid 0.53g (1.9) | BA 7.7(0.105) | 18 | ACN;15 | 0.127 | 30°C 312hr | 0.35g (76%) |
| 2 | DL-(Z)diaminobutyric acid 1g (3.6) | BA 8.21(0.11) | 32 | ACN;20 | 0.18 | 30°C 307r | 0.754g (90%) |
| 3 | DL-(Z)diaminobutyric acid 1g (3.6) | BA 4.38(0.06) | 60 | ACN;19 | 0.19 | 30°C 307r | 0.773g (91%) |
| 4 | DL-(Z)diaminobutyric acid 0.705g (2.53) | BA 2.22(0.03) | 83 | ACN;20 | 0.127 | 30°C 312hr | 0.491g (83%) |
| 5 | DL-(Z)diaminobutyric acid 0.705g (2.53) | BA 0.88(0.012) | 208 | ACN;20 | 0.127 | 30°C 312hr | 0.539g (91%) |
| 6 | DL-(Z)diaminobutyric acid 1.0g (3.6) | BA 0.77(0.011) | 340 | ACN;19 | 0.19 | 30°C 360hr | 0.764g (91%) |
| 7 | DL-(Z)diaminobutyric acid 1.0g (3.6) | BA 0.60(0.008) | 440 | ACN;19 | 0.19 | 30°C 360hr | 0.739g (88%) |
| 8 | DL-(Z)diaminobutyric acid 0.78g (2.8) | PEG1000 60(0.06) | 46 | ACN;175 | 0.19 | 30°C 307hr | 0.559g (76%) |

a) BA ; butylamine
PEG1000 ; polyethylene glycol MW 1000
b) N-Carboxy Anhydride (NCA) / Initiator mol ratio
c) ACN ; Acetonitrile

【table 2】

Synthetic Conditions for Poly(DL-2,4-diaminobutyric acid) Acetate by Deprotection of Poly(4-N-carbobenzoxy-DL-2,4-diaminobutyric Acid)

| Synthetic Example | Component a) (g) | TFA-HBrHAc (ml) | Temperature Time | NaOAc(g) $H_2O$(m) | Dialysis Time | Centrifugation Temperature | Yield | Number of Residue | Molecular Weight |
|---|---|---|---|---|---|---|---|---|---|
| 1 | Poly(Dba(Z)) 0.306 | 1.3-1.3 | 24°C 1.8hr | 0.31g 20ml | 500cut 17hr | 30,000rpm 4°C, 1hr | 0.115g (46%) | 12 | 1,900 |
| 2 | Poly(Dba(Z)) 0.509 | 2-2 | 22°C 2hr | 0.5g 25ml | 1000cut 17hr | 30,000rpm 4°C, 1hr | 0.267g (77%) | 26 | 4,200 |
| 3 | Poly(Dba(Z)) 0.506 | 2-2 | 22°C 2hr | 0.5g 25ml | 1000cut 17hr | 30,000rpm 4°C, 1hr | 0.339g (98%) | 49 | 7,800 |
| 4 | Poly(Dba(Z)) 0.352 | 1.5-1.5 | 24°C 2hr | 0.35g 25ml | 1000cut 17hr | 30,000rpm 4°C, 1hr | 0.139g (58%) | 62 | 9,900 |
| 5 | Poly(Dba(Z)) 0.379 | 1.5-1.5 | 24°C 1.8hr | 0.35g 25ml | 1000cut 17hr | 30,000rpm 4°C, 1hr | 0.195g (75%) | 170 | 27,200 |
| 6 | Poly(Dba(Z)) 0.379 | 1.5-1.5 | 24°C 1.8hr | 0.35g 25ml | 1000cut 17hr | 30,000rpm 4°C, 1hr | 0.210g (81%) | 278 | 44,500 |
| 7 | Poly(Dba(Z)) 0.379 | 1.5-1.5 | 24°C 1.8hr | 0.35g 25ml | 1000cut 17hr | 30,000rpm 4°C, 1hr | 0.207g (80%) | 348 | 55,700 |
| 8 | Poly(Dba(Z)) -PEG 0.416 | 2-2 | 22°C 2hr | 0.5g 25ml | 1000cut 17hr | 30,000rpm 4°C, 1hr | 0.254g (78%) | 31 | 6,000 |

a)

Poly(Dba(Z)) ; Poly(4-N-Carbobenzoxy-DL-2,4-diaminobutyric acid)

Poly(Dba(Z))-PEG ; Poly(4-N-Carbobenzoxy-DL-2,4-diaminobutyric acid)-PEG100

(EXAMPLE 2) Gene introduction into HepG2 cells with pDBAs having a variety of molecular weights

Preparation of plasmid/pDBA complexes:

A solution of a plasmid encoding the luciferase gene (about 5.25 kb: PicaGene control vector into which the luciferase gene was previously introduced (Toyo Ink Mfg. Co. Ltd.) that will be referred to as "plasmid A" hereinafter) and a solution of a nucleic acid carrier were prepared, respectively, at twice the desired concentration. Approximately 30 minutes prior to administration, the nucleic acid carrier solution was added dropwise to the plasmid solution with stirring to prepare a plasmid/(nucleic acid carrier) complex solution. DMEM medium (Sigma-Aldrich Corporation) was used as solvent. Specifically, a 25 μg/ml plasmid solution was prepared such that a plasmid/(nucleic acid carrier) complex solution having a plasmid concentration of 12.5 μg/ml would be used as an administration sample.

Administration of plasmid/(nucleic acid carrier) complex solution to HepG2 cells and method of measurement:

HepG2 cells were inoculated onto a 12-well multi-well plate (Coaster Inc.) at $1x10^5$ cells/well one day prior to testing. Administration of the plasmid/(nucleic acid carrier) complex solution was carried out in the presence of 10% fetal bovine serum (Sanko Junyaku Co. Ltd.) (with a final concentration of the plasmid being 2.5 μg/ml) and incubation was carried out at 37 °C for 4 hours. Medium was exchanged with a fresh culture medium and incubation continued for 48 hours. After washing with PBS twice, a cell lysis reagent (Toyo Ink Mfg. Co. Ltd.) was added and a freeze-thawing cycle was conducted once. The cell lysate was recovered and centrifuged (12,000rpm x 10 minutes). The luciferase activity of the supernatant was measured on a Luciferase Assay System (Toyo Inc. Mfg. Co. Ltd.) using a luminometer (Lumit LB9501 available from Berthold Pty Ltd.). The protein concentration of the centrifuged supernatant was measured in a Protein Assay Kit (Bio-Rad Laboratories, Inc.) using a microplate reader (Rainbow Thermo available from Tecan U.S., Inc.).

pDBAs were used that had been obtained in Synthetic Examples 1, 2, 3, 4, 5, and 7 (all listed in Table 2). Complex solutions with the weight ratio of plasmid/pDBA being 1/5 (w/w) were prepared and used in testing. The results from measurements of the activity of the expressed luciferase are shown in Figure 2. It is evident that the greater the number of residues (i.e., the greater the molecular weight of pDBA), the higher the gene expression. This has confirmed that the preferred pDBA is one that has a degree of polymerization of greater than a certain level.

(EXAMPLE 3) Gene introduction into HepG2 cells with complexes where the weight ratio of pDBA to plasmid is varied.

Similarly to Example 2, gene introduction into HepG2 cells was investigated. pDBA obtained in Synthetic Example 3 and pDBApeg obtained in Synthetic Example 8 were used. Complex solutions were prepared for testing such that the weight ratios of plasmid to pDBA (plasmid/pDBA) were 1/1, 1/3, 1/5, 1/7, 1/10, and 1/20 (w/w), respectively. In the case of pDBApeg, complex solutions were prepared for testing such that these ratios were 1/1, 1/5, and 1/10 (w/w), respectively.

The results from measurements of the activity of the expressed luciferase are shown in Fig. 3; as the quantity of pDBA relative to plasmid increases, so does the gene expression.

(EXAMPLE 4) Gene introduction into HepG2 cells with pDBA using a large size plasmid

The luciferase gene was inserted into the multicloning site of a plasmid which would encode the luciferase gene like plasmid A used in Examples 1-2 and which was larger in size than plasmid A (about 11.1 kb, EBV vector (pREP7 available from Funakoshi Co. Ltd.)) according to the conventional gene manipulation method (Sambrook et al., Molecular Cloning, A Laboratory Manual, 2nd ed., Cold Spring Harbor laboratory Press, 1989): this plasmid will be abbreviated as "plasmid B" hereinafter. To confirm the gene introduction, gene introduction into HepG2 cells was investigated using a method similar to Example 1.

Five kinds of pDBAs used were those obtained in Synthetic Examples 1, 2, 3, 4, and 5 (all listed in Table 2). Complex solutions were prepared for use in testing such that the weight ratios of plasmid B to pDBA were 1/5 and 1/10 (w/w), respectively.

The results from measurements of the activity of the expressed luciferase are shown in Fig. 4. It has been shown that pDBA can make the gene to express with the large size plasmid (11.1 kb) similarly to the plasmid (5.25 kb) used in Example 2.

These results indicate that the introduction of the therapeutic gene using the nucleic acid carrier of this invention is not limited by the size of said gene.

(EXAMPLE 5) Gene introduction into mice using pDBA complexes.

Preparation of plasmid/pDBA complexes:

A solution of plasmid A and a solution of pDBA were prepared, respectively, at twice the desired concentration. Approximately 30 minutes prior to administration, the pDBA solution was added dropwise to the plasmid solution with stirring to prepare a plasmid/(nucleic acid carrier) complex solution. DMEM medium was used as solvent. Specifically, a 50 μg/ml plasmid solution was prepared such that a plasmid/(nucleic acid carrier) complex solution having a plasmid concentration of 25.0 μg/ml would be used as an administration sample.

Complex solutions were prepared from pDBA obtained in Example 3 and plasmid A such that the weight ratio of plasmid/pDBA was 1/7 (w/w) and were administered to Balb/c mice intravenously through tail veins. The dose of plasmid A per mouse was 12.5 μg/0.5 ml. Two days and twenty one days after administration the luciferase activity was measured in the lung, the liver and the spleen whereby gene introduction to the respective were determined.

The results obtained are shown in Fig. 5. As Fig. 5A shows, in the group where pDBA was used as the nucleic acid carrier, the liver exhibited markedly high luciferase activity two days after administration when measured as compared with the group where only the plasmid was administered. These results indicate that when systemic administration is carried out using the nucleic acid carrier of this invention, it is possible to specifically introduce the gene into the liver. Further, these results indicate that it is also possible to introduce the gene into a specific tissue by systemically administering it through optimization of the gene carrier of this invention. As Fig. 5B shows, the luciferase activity in the liver was maintained even 21 days after administration. This result indicates that when systemic administration is carried out using the nucleic acid carrier of this invention, it is possible to prolong the gene expression in a specific tissue. Furthermore, these results indicate that it will be possible to optimize the duration of expression of the specific gene in a specific tissue by systemically administering it through optimization of the nucleic acid carrier of this invention.

(EXAMPLE 6) Gene introduction into mice with complexes where the weight ratio of pDBA to plasmid is varied

Similarly to Example 4, gene introduction with the complexes of plasmid A and pDBA that were prepared at different weight ratios was assessed by manifestation of the luciferase activity.

Complex solutions were prepared from pDBA obtained in Synthetic Example 3 and plasmid A such that different weight ratios of plasmid to pDBA (plasmid/pDBA=1/0, 1/1, 1/5, 1/10, and 1/20) were realized; and they were administered to Balb/c mice intravenously through tail veins.

Two days after administration the luciferase activity found in the liver was measured whereby gene introduction to the respective mouse tissues were determined.

The results obtained are shown in Fig. 6. As the quantity of pDBA relative to the plasmid increases, the gene expression has the tendency to increase, which has been clearly identified.

(EXAMPLE 7) Gene introduction into mice with pDBAs having various molecular weights

Similarly to Example 4, gene introduction with the complexes of plasmid A and pDBA that were prepared was assessed by manifestation of the luciferase activity.

Complexes were prepared from five kinds of pDBA obtained in Synthetic Examples 1, 2, 3, 4, and 5 (all listed in Table 2) and plasmid A such that the weight ratio of plasmid/pDBA was 1/5 (w/w); and they were administered to mice intravenously through tail veins. Two days after administration the luciferase activity in the liver was measured.

The results obtained are shown in Fig. 7. As the number of residues of pDBA increases, the gene expression has the noted tendency to increase. As with the results of Example 5, it has been confirmed that the degree of polymerization for diaminobutyric acid is preferably over a certain level.

(EXAMPLE 8) Duration of expression of the gene intravenously administered in mice through tail veins

Similarly to Example 4, Plasmid A/pDBA complex solutions that were prepared were administered to Balb/C mice (8-week old male) intravenously through tail veins. Gene expression in the liver after administration was assessed by measuring the luciferase activity. pDBA obtained in Synthetic Example 3 in Table 2 was used to prepare a complex solution such that plasmid/pDBA was equal to 1/5 (w/W), which was used in the administration described above.

The results obtained are shown in Fig. 8. In the group where the plasmid/pDBA complex was administered, the gene expression could be confirmed from the second day of administration till 7 months later. Namely, it was ascertained that a single administration could cause the gene to express in the liver over 7 months. For comparison, administration of only the plasmid as well as administration of a complex of the plasmid with ExGen500 (Euromedex Inc.) which is a commercial gene introduction reagent was carried out. The results obtained are shown in Fig. 8. In

both cases, gene expression was low as compared to the group administered with the plasmid/pDBA: the gene expression was no longer detectable within one month in the administration of only the plasmid, and within three months in the group administered with the complex of ExGen500 with the plasmid.

These results indicate that it will be possible to optimize the duration of expression of the specific gene in a specific tissue by optimizing the nucleic acid carrier of this invention.

(EXAMPLE 9) Survival rate of mice singly administered with high doses of pDBA

The survival rate of mice was investigated in the manner described below when high doses of pDBA solution were directly administered to the mice.

pDBA was dissolved in physiological saline solution to prepare pDBA solutions having different concentrations. The pDBA solutions were administered to Balb/c mice intravenously through tail veins, and their symptoms were observed. pDBAs obtained in Synthetic Examples 1-7 (all listed in Table 2) were used. Results are shown in Table 3.

Table 3

| Dose (mg/kg) | Survival rate (survival number/case number) | | | | | | |
|---|---|---|---|---|---|---|---|
| | AA residue of pDBA | | | | | | |
| | 12 | 26 | 49 | 62 | 170 | 278 | 348 |
| 12.5 | 0/3 | 0/3 | 0/3 | 0/3 | - | - | - |
| 6.3 | 3/3 | 3/3 | 3/3 | 2/3 | 0/3 | 0/3 | 0/3 |
| 3.1 | 3/3 | 3/3 | 3/3 | 3/3 | 3/3 | 3/3 | 1/3 |
| 1.6 | 3/3 | 3/3 | 3/3 | 3/3 | 3/3 | 3/3 | 3/3 |
| "-": not done | | | | | | | |

Number of amino acid residues, number of synthetic examples, molecular weight:
12, Synthetic Example 1, MW 2,000; 26, Synthetic Example 2, MW 4,200; 49, Synthetic Example 3, MW 7,900; 62, Synthetic Example 4, MW 9,900; 170, Synthetic Example 5, MW 27,200; 278, Synthetic Example 6, MW 44,500; 348, Synthetic Example 7, MW 55,700

For a comparison purpose, the survival rate of mice that were subjected to the single administration of high doses of polyethyleneimine was investigated in a similar manner to that described above. In this instance, polyethyleneimine (PEI)(Gene Therapy, 4, 1100, 1997), which is the principal ingredient of ExGen500, and a cationic polymer, was used. PEI with a molecular weight of 1,800 and PEI with a molecular weight of 10,000 (both available from Wako Pure Chemical Industries, Co. Ltd.) were diluted with physiological saline solution for use. Comparative results are shown in Table 4.

Table 4

| Dose (mg/kg) | Survival rate (survival number/case number) | |
|---|---|---|
| | PEI (polyethyleneimine) | |
| | MW 1,800 | MW 10,000 |
| 12.5 | 0/1 | 0/3 |
| 9.3 | 1/3 | - |
| 6.1 | 2/3 | 0/3 |
| 3.1 | - | 0/3 |
| 1.5 | - | 2/3 |

From Tables 3 and 4, it is found that the safety of pDBA is higher than that of PEI. When PEI was administered as a single entity, it was observed that the high dose administration of PEI with large molecular weights would likely cause disorders of internal organs in the digestive system and that PEI with small molecular weights would likely cause hemorrhage in the lung.

While the safety of pDBA is considered to be at the same level as other cationinc polyamino acids, it is higher than polyethyleneimine (PEI), the comparative example. As Examples 3 and 6 show, those with higher degrees of

polymerization of amino acid are preferred in terms of efficacy; however, the higher the degree of polymerization, more likely is it believed to cause disorders in the liver by administration at high doses. In consideration of the effects and the safety, there should be a preferred range for the number of residues of pDBA that serves as the nucleic acid carrier: it is believed to be from 10 to 280; and further the most preferred range is believed to be from 20 to 280.

(EAMPLE 10) Antigenicity tests of pDBA using guinea pigs

Sensitization of animals:

Male guinea pigs (Hartley line) were sensitized by being subcutaneously administered pDBA (obtained in Synthetic Example 3) once a week over 4 weeks such that the administration provided 1 mg/kg. Five days after the final administration, the animals were anesthetized with ether and blood was collected from their hearts. The obtained sera were used to carry out a 4-hour passive cutenous anaphylaxis test, where non-treated guinea pigs were also used. The sensitized guinea pigs were subjected to an active systemic anaphylaxis test three days after blood collection. Animals were sensitized by being administered bovine serum albumin (BSA) at a level of 10 mg/kg as a positive control group.

Confirmation of antigenicity by active systemic anaphylaxis:

Eight days after the final administration, a test solution (pDBA, 1 mg/kg) was administered to each animal at 0.1 ml/100 g through the vein of the auricle or the vein of the inner side of the forefoot. The BSA-administered group, which was a positive control, was treated similarly and the BSA solution was intravenously administered (10 mg/kg) to the group. The symptoms appearing within one hour after intravenous administration were observed according to the criteria shown below. The results obtained are shown in Fig. 9. The immunogenicity of pDBA could be confirmed to be low. Judging criteria for the active systemic anaphylaxis are shown below.

| Symptoms | Evaluation scores |
|---|---|
| no change | 0 |
| piloerection, nose scratching, anxiety | 1 |
| trembling, sneeze, hyperponesis (in addition to those mentioned above) | 2 |
| urination, defecation, dyspnea (in addition to those mentioned above) | 3 |
| convulsion, fall (in addition to those mentioned above) | 4 |
| death | 5 |

Confirmation of antigenicity by 4-hour passive cutaneous anaphylaxis:

Portions (each 0.1 ml) of the original sera derived from the sensitized animals and the sera diluted with physiological saline (10-, 100-, 1000-,and 10000-fold) were subcutaneously administered to unsensitized guinea pigs that had been shaven at their back skin. A sample solution (pDBA, 1 mg/kg) containing 0.5% Evans Blue was administered to the vein of the auricle or the vein of the inner side of the forefoot at 0.1 ml/100 g. The BSA-administered group, which was the positive control, was treated similarly and intravenous administration (1 mg/kg) of a BSA solution containing 0.5% Evans Blue was carried out. The diameters of pigment maculae appearing on the skin were measured. The results obtained are shown in Fig. 10. In the BSA group, which was the positive control, cutaneous reaction was detected even when the sera were diluted from 100-fold to 1000-fold; in contrast, in the pDBA group cutaneous reaction was not induced even when the original serum was administered. This was similar to the results of systemic anaphylaxis. It was then shown that the immunogenicity of pDBA was very low.

(EXAMPLE 11) Introduction into cells of an oligonucleotide using pDBA

An oligonucleotide labeled with FITC (20mer, Pharmacia Corporation) was used to assess the efficiency of introduction into cells for the complexes of the oligonucleotide with various pDBAs.

HepG2 cells were inoculated onto a 12-well petri dish at $1x10^5$ cells/well and cultured for 24 hours. The FITC-labeled oligonucleotide was used, and pDBAs obtained in Synthetic Examples 3 and 5 were used as the nucleic acid carriers. Complexes were prepared from these three kinds (pDBAs and the FITC-labeled oligonucleotide) such that the ratios of oligonucleotide/pDBA were 1/1, 1/5, and 1/10 (w/w). The complex solutions were added to the HepG2 cells; and after culturing for 4 hours, the cells were washed with PBS(-) twice. After removal of PBS(-), the cells were

separated from the dish using a cell dissociation solution (SIGMA-Aldrich Corporation) and the cell suspensions were subjected to measurement with a flow cytometer (FACS Calibur available from Becton Dickinson). The results are shown in Fig. 11 (oligonucleotide/pDBA=1/5 (w/w)), where M1 represents the results from control experiment and M2 represents the results from the experiment using the FITC-labeled oligonucleotide). Fig. 12 shows the results that are assessed by the proportions of cells into which the FITC-labeled oligonucleotide was introduced among 10,000 cells (oligonucleotide/pDBA=1/1, 1/5, 1/10(w/w)).

From Fig. 11 it has been confirmed that with only the oligonucleotide cellular introduction hardly takes place. The proportions of cells into which the FITC-labeled oligonucleotide had been introduced were calculated from Fig. 12. With only the oligonucleotide, cellular introduction hardly took place; and there was little incorporation of oligonucleotide in the group where pDBA obtained in Synthetic Example 1 (the number of residues=12) was used as a nucleic acid carrier. By contrast, in the group of the complexes with pDBAs obtained in Synthetic Example 3 (the number of residues=49) and Synthetic Example 5 (the number of residues=170), where the ratio of oligonucleotide/pDBA was equal to or greater than 1/5 (w/w), incorporation of the FITC-labeled oligonucleotide was observed in about 70-90% of cells among the 10,000 cells subjected to the measurement.

These results have confirmed that the nucleic acid carrier of this invention can preferably be used for oligonucleotides such as antisense oligonucleotides and TFO (Triplex Forming Oligonucleotides).

**Industrial Applicability**

The nucleic acid carriers of this invention and a variety of therapeutic genes can form complexes (pharmaceutical compositions of this invention) that are safe and have extremely low immunogenicity; and they can allow the therapeutic genes to be introduced into cells efficiently and safely, as well as can allow for high expression of the genes in the cells. By selecting a suitable number of residues, it will also be possible to apply genes of various sizes and a wide variety of therapeutic genes (including oligonucleotides such as antisense oligonucleotides and TFO (Triplex Forming Oligonucleotide)) for any expression.

Further, the nucleic acid carriers of this invention are used to systemically administer the therapeutic genes whereby the genes can be specifically introduced into the liver.

Still further, it will be possible to introduce the genes into a specific tissue by systemically administering them through optimization of the nucleic acid carriers of this invention. Furthermore, it will be possible to optimize the duration of expression of the specific genes in a specific tissue, including the prolongation of their expression.

**Claims**

1. A nucleic acid carrier comprising a polypeptide comprising diaminobutyric acid and/or a pharmaceutically acceptable salt thereof.

2. A nucleic acid carrier comprising a block copolymer of a polypeptide comprising diaminobutyric acid and/or a pharmaceutically acceptable salt thereof and polyethylene glycol.

3. The nucleic acid carrier according to claim 1 or 2, wherein the peptide comprises diaminobutyric acid and/or a pharmaceutically acceptable salt thereof, each having a residue number of from 20 to 280.

4. A pharmaceutical composition for gene therapy comprising the nucleic acid according to claim 1 or 2 and a therapeutic gene.

5. The pharmaceutical composition for gene therapy according to claim 4, wherein the therapeutic gene is a nucleic acid and/or a nucleotide derivative.

6. A pharmaceutical composition for gene therapy comprising the nucleic acid according to claim 3 and a therapeutic gene.

7. The pharmaceutical composition for gene therapy according to claim 6, wherein the therapeutic gene is a nucleic acid and/or a nucleotide derivative.

*Fig.1*

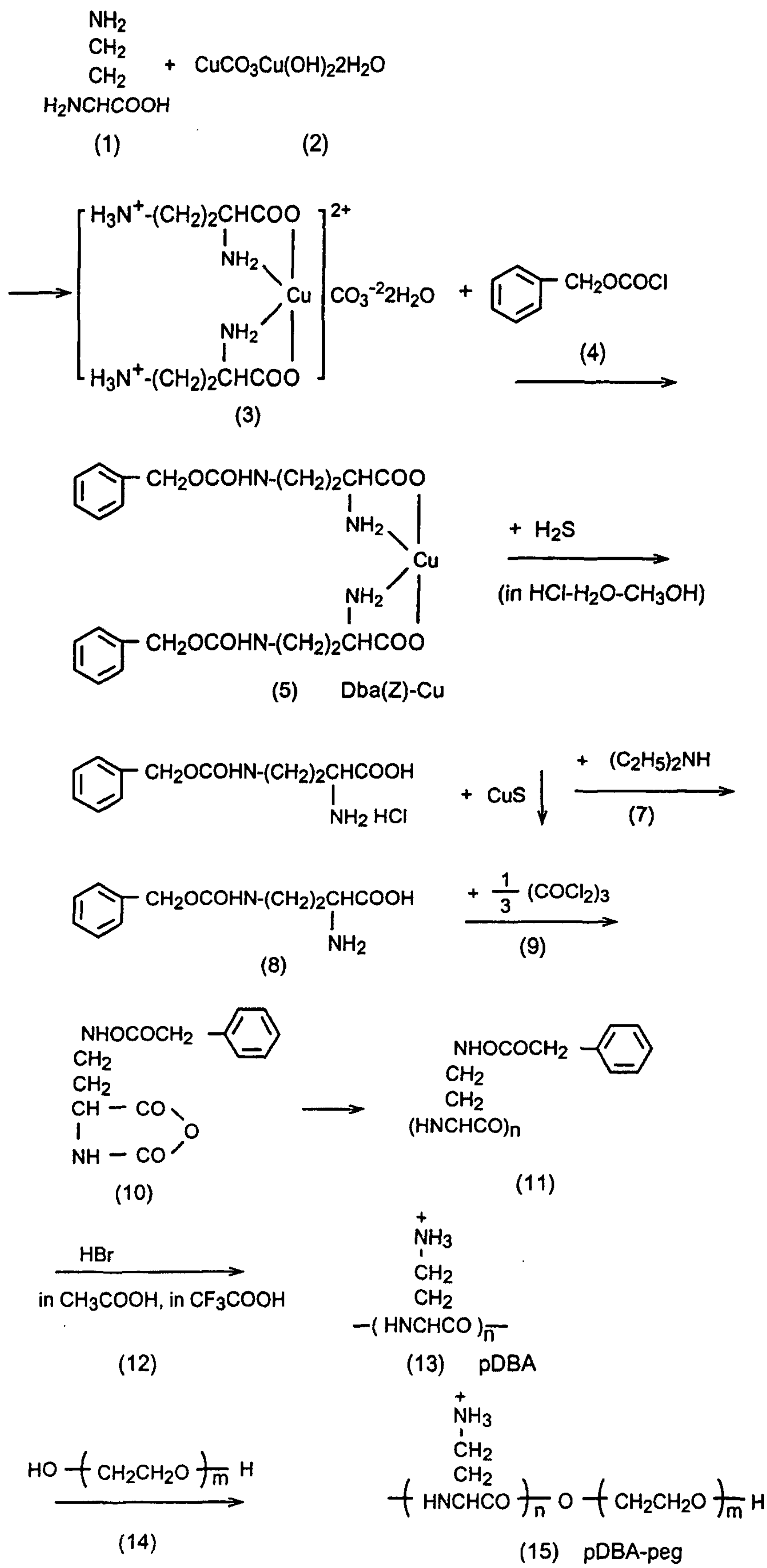


(1) (2) (3) (4) (5) Dba(Z)-Cu (7) (8) (9) (10) (11) (12) (13) pDBA (14) (15) pDBA-peg

*Fig.2*

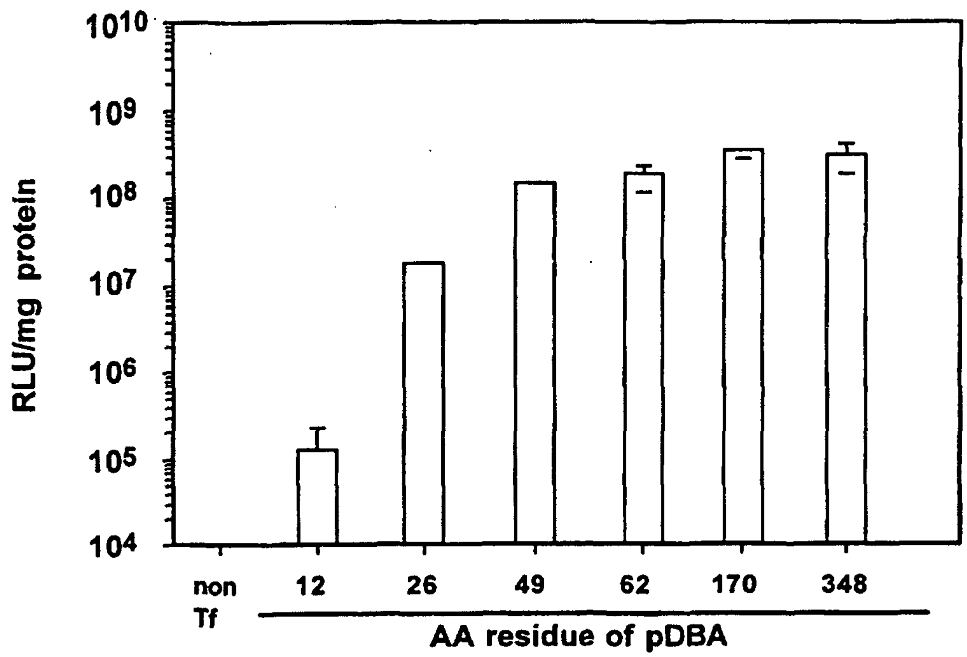
10^10
10^9
10^8
10^7
10^6
10^5
10^4
RLU/mg protein
non Tf
12
26
49
62
170
348
AA residue of pDBA

*Fig.3*

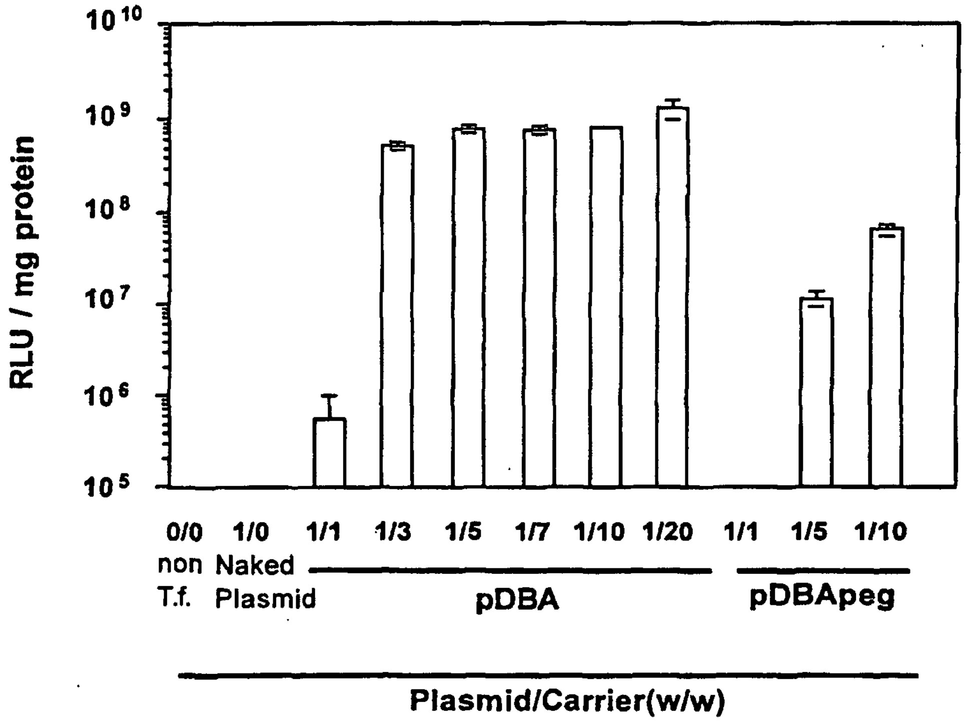
10^10
10^9
10^8
10^7
10^6
10^5
RLU / mg protein
0/0 1/0 1/1 1/3 1/5 1/7 1/10 1/20 1/1 1/5 1/10
non T.f.
Naked Plasmid
pDBA
pDBApeg
Plasmid/Carrier(w/w)

*Fig.4*

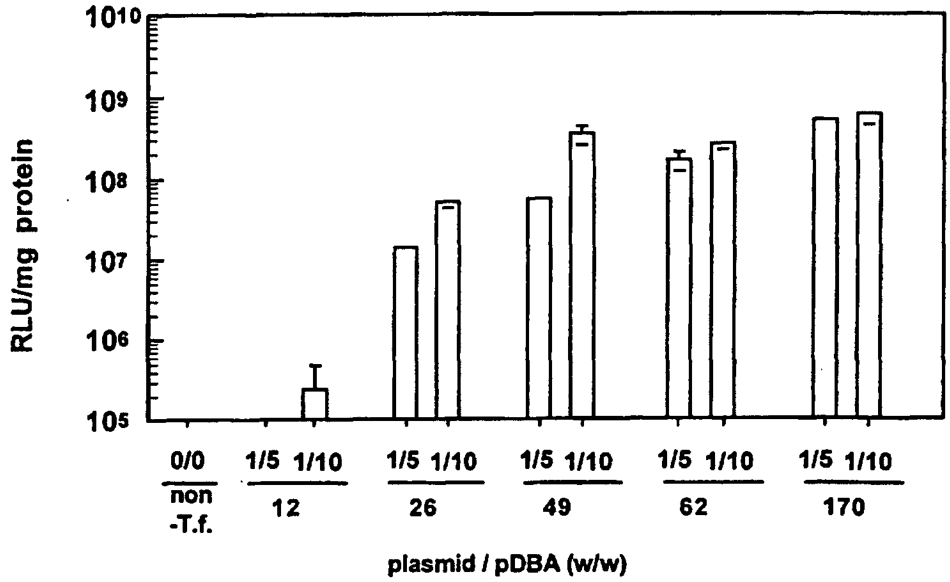
10^10
10^9
10^8
10^7
10^6
10^5
RLU/mg protein
0/0
non
-T.f.
1/5 1/10
12
1/5 1/10
26
1/5 1/10
49
1/5 1/10
62
1/5 1/10
170
plasmid / pDBA (w/w)

Fig.5A day2

Fig.5B day21

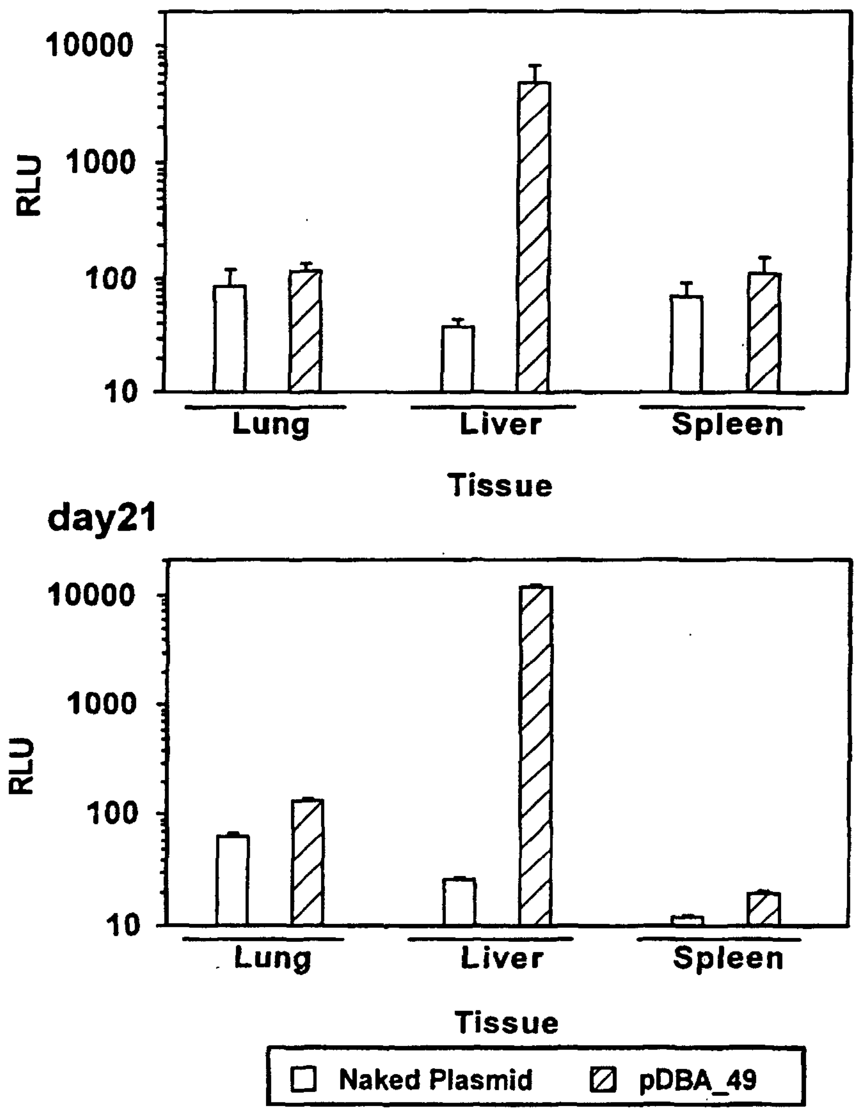


Balb/C mouse (i.v.)
Mean±S.E.
(n=3)

*Fig.6*

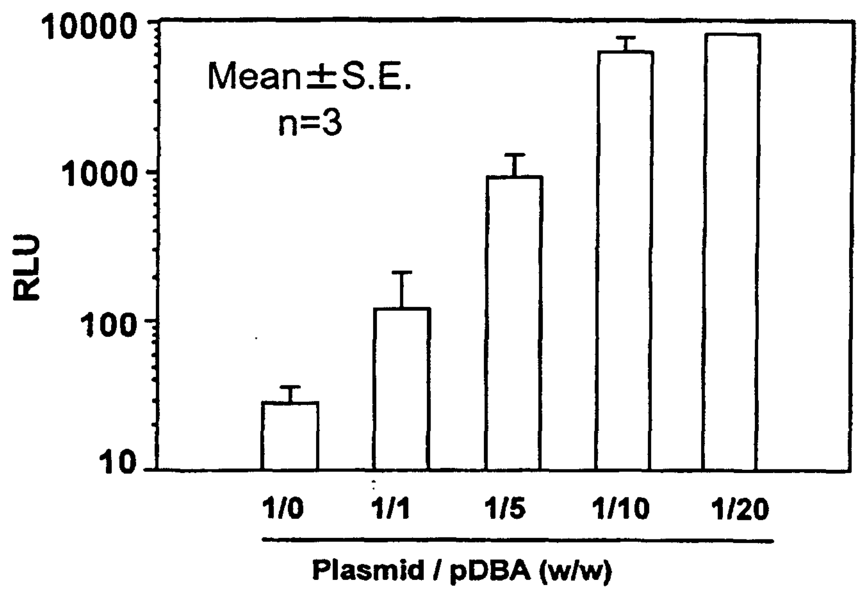


1/0; Naked Plasmid

*Fig.7*

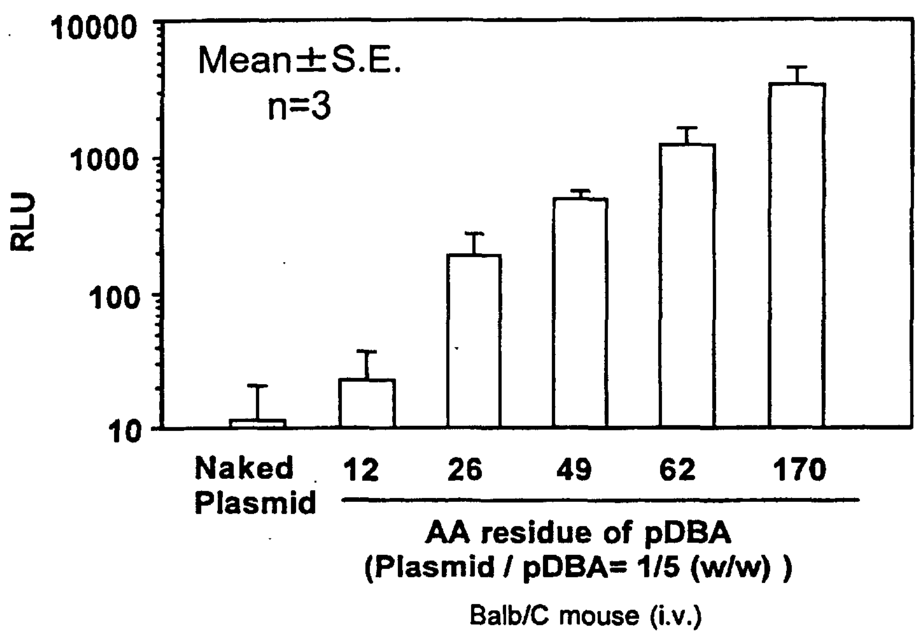
10000
1000
100
10
RLU
Mean±S.E.
n=3
Naked Plasmid
12
26
49
62
170
AA residue of pDBA
(Plasmid / pDBA= 1/5 (w/w) )
Balb/C mouse (i.v.)

**Fig.8**

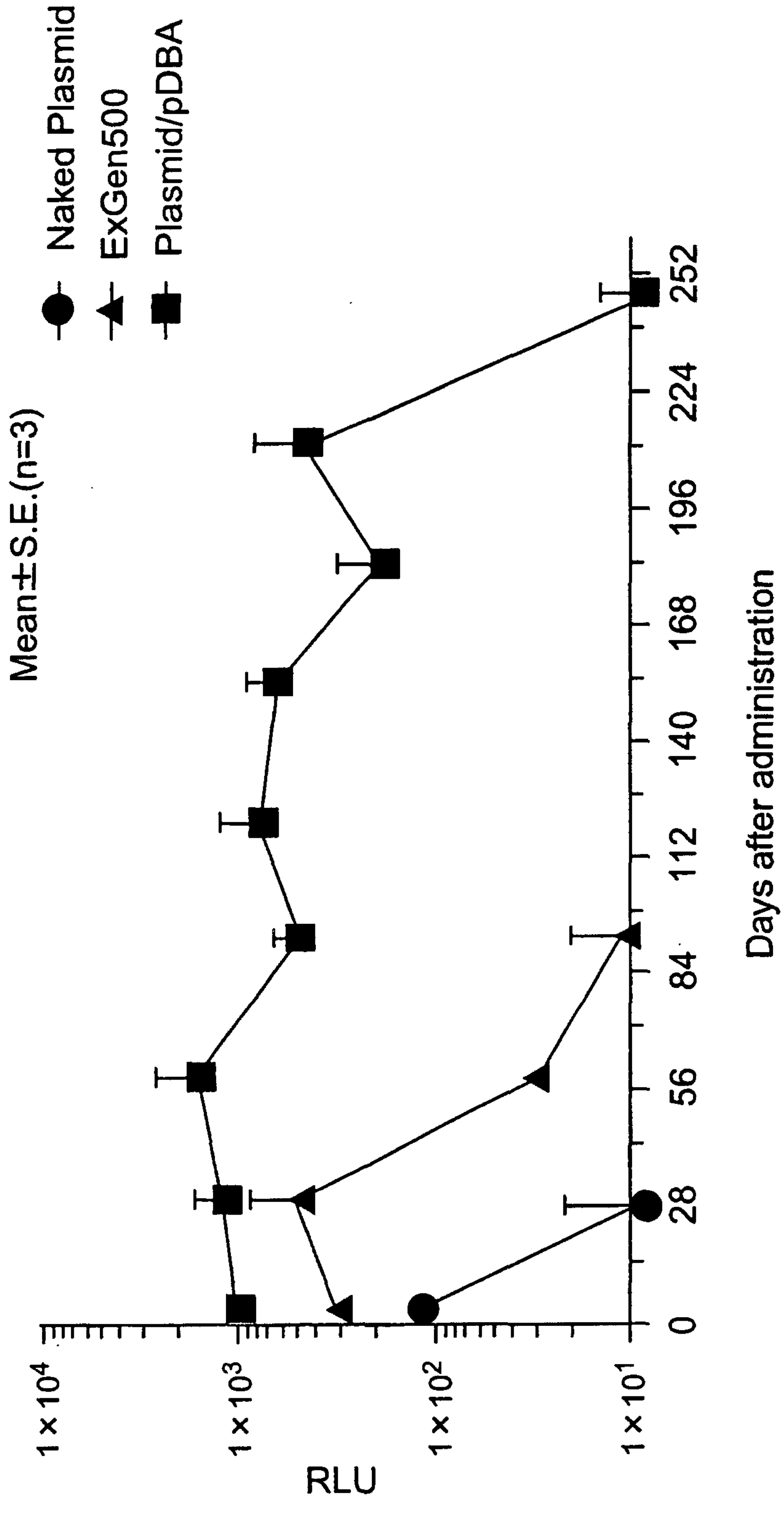
Mean±S.E.(n=3)
Naked Plasmid
ExGen500
Plasmid/pDBA
RLU
1×10⁴
1×10³
1×10²
1×10¹
0
28
56
84
112
140
168
196
224
252
Days after administration

*Fig.9*

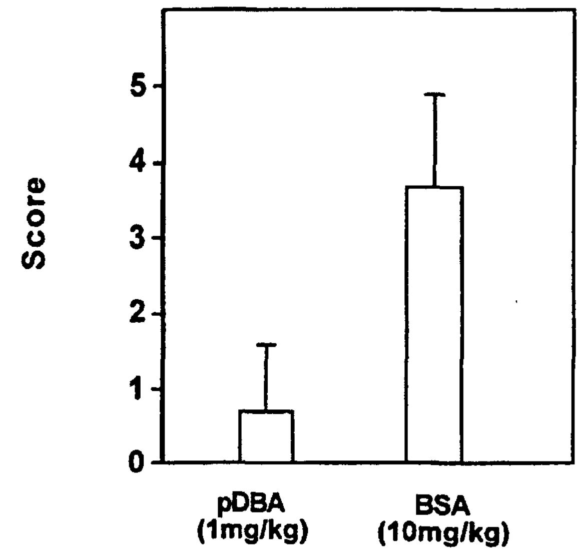


Active systemic anaphylaxis in guinea pigs
n=3

*Fig.10*

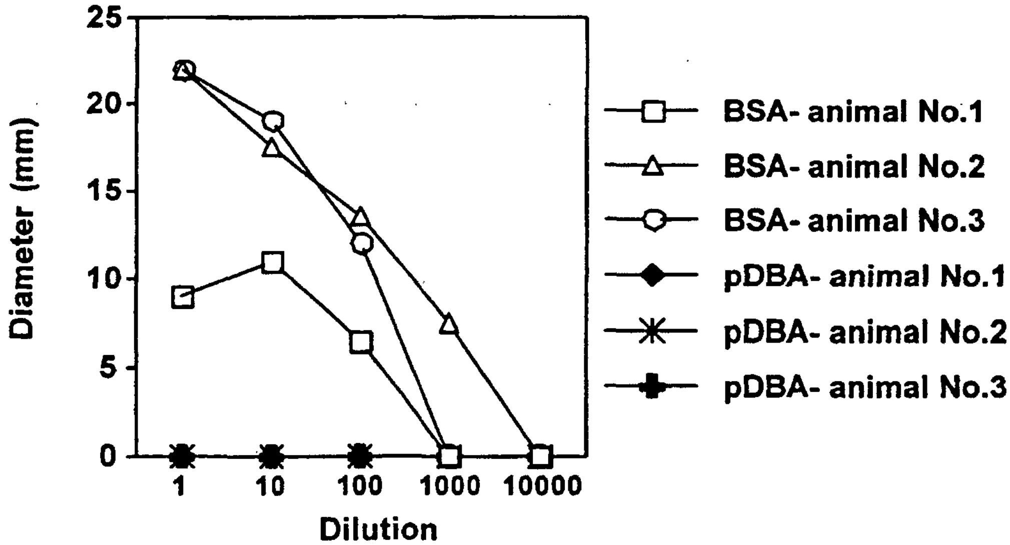


Passive cutaneous anaphylaxis in guinea pigs with sera obtained from sensitized guinea pigs

Fig.11A

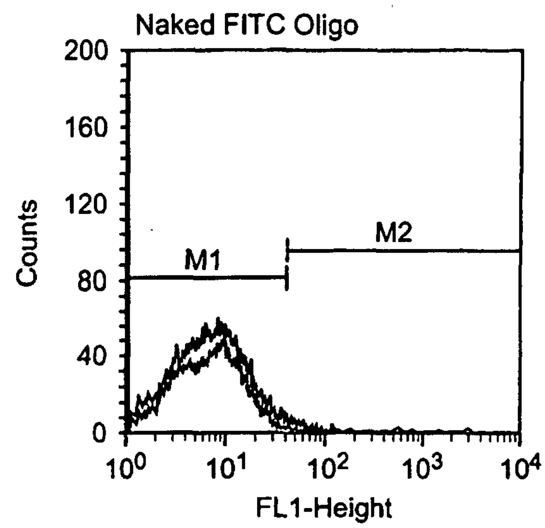


Fig.11B

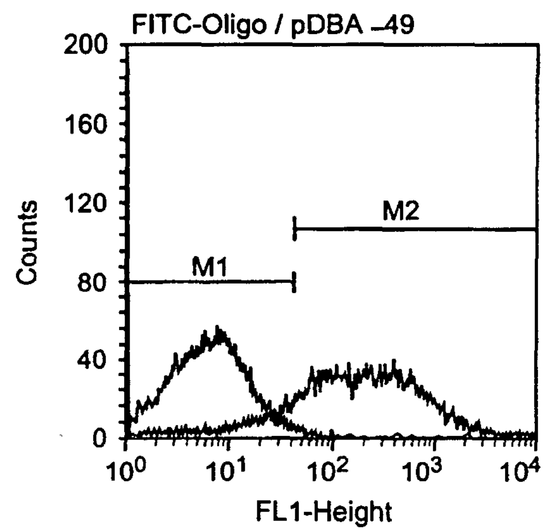


Fig.11C

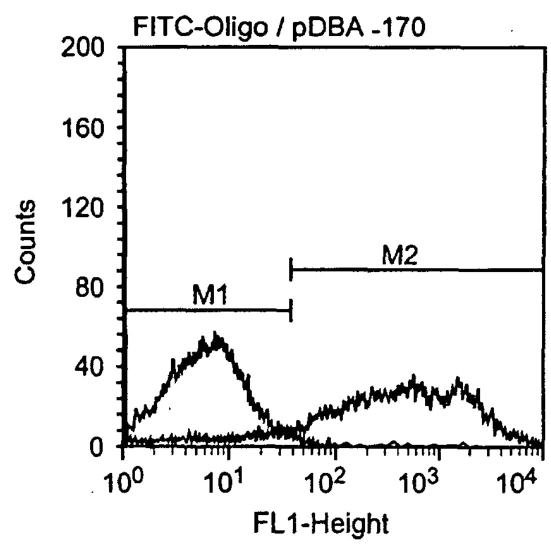

# Fig.12

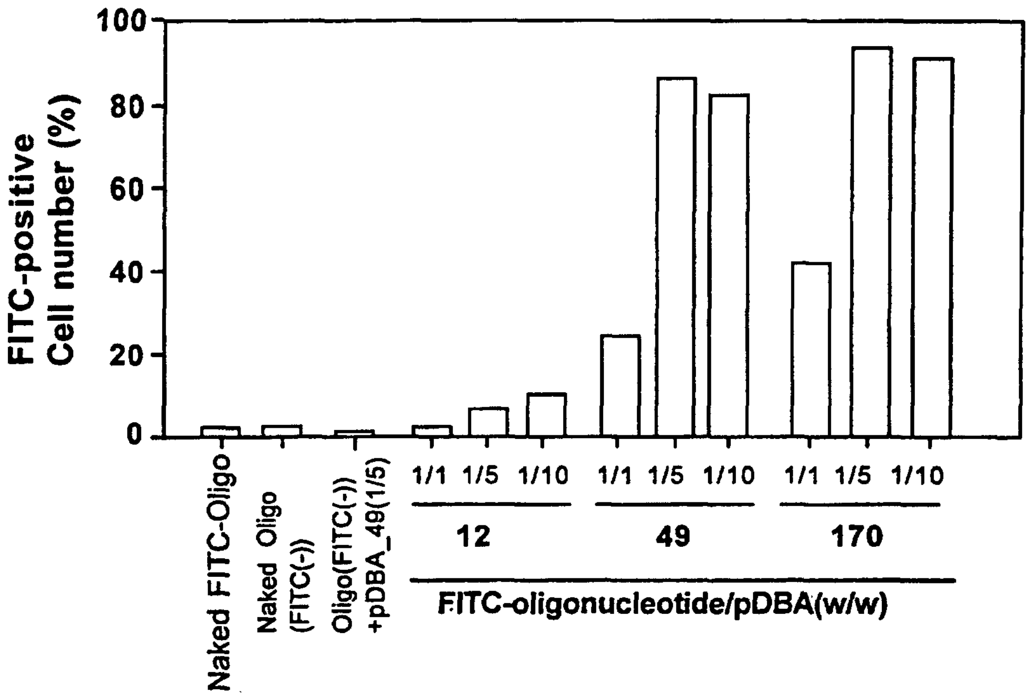
FITC-positive
Cell number (%)
100
80
60
40
20
0
Naked FITC-Oligo
Naked Oligo
(FITC(-))
Oligo(FITC(-))
+pDBA_49(1/5)
1/1 1/5 1/10
12
1/1 1/5 1/10
49
1/1 1/5 1/10
170
FITC-oligonucleotide/pDBA(w/w)

## INTERNATIONAL SEARCH REPORT

International application No.
PCT/JP99/06415

A. CLASSIFICATION OF SUBJECT MATTER
Int.Cl$^7$ A61K48/00

According to International Patent Classification (IPC) or to both national classification and IPC

B. FIELDS SEARCHED

Minimum documentation searched (classification system followed by classification symbols)
Int.Cl$^7$ A61K48/00

Documentation searched other than minimum documentation to the extent that such documents are included in the fields searched

| | | | |
|---|---|---|---|
| Jitsuyo Shinan Koho | 1940-1992 | Toroku Jitsuyo Shinan Koho | 1994-1996 |
| Kokai Jitsuyo Shinan Koho | 1971-1992 | Jitsuyo Shinan Toroku Koho | 1996-1999 |

Electronic data base consulted during the international search (name of data base and, where practicable, search terms used)
CAPLUS(STN),EMBASE(STN),REGISTRY(STN),MEDLINE(STN),BIOSIS(STN)

C. DOCUMENTS CONSIDERED TO BE RELEVANT

| Category* | Citation of document, with indication, where appropriate, of the relevant passages | Relevant to claim No. |
|---|---|---|
| X | EP, 834572, A2 (F. HOFFMANN-LA ROCHE AKTIENGESELLSCHAFT), 08 April, 1998 (08.04.98), Full text; especially, Claims, P.3, lines 32-36; P.3, lines 42-50 & JP, 10-147595, A Full text; especially, Claims, Par. Nos. [0018], [0021], [0022] & CA, 2213257, A & BR, 9704955, A | 1-7 |
| A | EP, 775751, A2 (F. HOFFMANN-LA ROCHE AKTIENGESELLSCHAFT), 28 May, 1997 (28.05.97) & JP, 9-173067, A & CA, 2190706, A & US, 5759827, A | 1-7 |
| A | Jean-Yves et al."N-Acyl-(α,γ-Diaminobutyric Acid)n Hydrazide as an Efficient Gene Transfer Vector in Mammalian Cells in Culture", Pharmaceutical Research, Vol.14, No.5 (1997) P.619-624 | 1-7 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 February, 2000 (07.02.00) | 22 February, 2000 (22.02.00) |
| Name and mailing address of the ISA/ Japanese Patent Office | Authorized officer |
| Facsimile No. | Telephone No. |

Form PCT/ISA/210 (second sheet) (July 1992)